# EUROPEAN PATENT SPECIFICATION

(11) **EP 2 111 234 B1**
(45) Date of publication and mention of the grant of the patent: **19.03.2014**
(21) Application number: 08779554.8
(22) Date of filing: 29.01.2008
(51) Int. Cl.: A61K 39/42, A61K 39/00, A61K 39/395, C12N 3/00, C12N 1/06

(54) **METHODS OF RELEASING SPOROCYSTS FROM OOCYSTS USING CONTROLLED SHEAR FORCES**
VERFAHREN ZUR FREISETZUNG VON SPOROZYSTEN AUS OOZYSTEN MITTELS GESTEUERTER SCHUBKRÄFTE
PROCEDES DE LIBERATION DE SPOROCYSTES CONTENUS DANS DES OOCYSTES AU MOYEN DE FORCES DE CISAILLEMENT REGULEES

(30) Priority: 08.02.2007 US 900233 P; 28.01.2008 US 20955
(43) Date of publication of application: 28.10.2009
(73) Proprietor: EMBREX Inc., Durham, NC 27703 (US)
(72) Inventor: HUTCHINS, James Earl, Durham, NC 27713 (US); WILSON, Kerrianne, Chester Springs, PA 19425 (US); HARTMAN, Angela, Durham, NC 27703 (US); HARRIS, Kelly Michelle, Wake Forrest, NC 27587 (US)
(74) Representative: Mannion, Sally Kim
(86) International application number: PCT/US2008/001142
(87) International publication number: WO 2008/118255

(56) References cited:
- US-A- 3 147 186
- US-A- 4 544 548
- US-A- 4 863 731
- US-A1- 2002 127 618
- US-A1- 2003 175 311
- US-A1- 2006 121 060
- DULSKI P ET AL: "THE PURIFICATION OF SPOROCYSTS AND SPOROZOITES FROM EIMERIA-TENELLA OOCYSTS USING PERCOLL DENSITY GRADIENTS" AVIAN DISEASES, AMERICAN ASSOCIATION OF AVIAN PATHOLOGISTS, KENNET SQ., PA, US, vol. 32, no. 2, 1 January 1988 (1988-01-01), pages 235-239, XP008127538 ISSN: 0005-2086
- P. COUDERT, D. LICOIS, F.DROUET-VIARD: "Eimeria species and strains of rabbits" BIOTECHNOLOGYGUIDELINES ON TECHNIQUES IN COCCIDIOSIS RESEARCH, 1995, - 1995 pages 52-73, XP008127605 Luxembourg
- HOSEK J E ET AL: "IMPROVED METHOD FOR HIGH-YIELD EXCYSTATION AND PURIFICATION OF INFECTIVE SPOROZOITES OF EIMERIA SPP" JOURNAL OF PROTOZOOLOGY, LAWRENCE, KANSAS, US, vol. 35, no. 4, 1 January 1988 (1988-01-01), pages 583-589, XP009034045 ISSN: 0022-3921

## Description

### FIELD OF THE INVENTION

The present invention relates to methods of releasing sporocysts from oocysts.

### BACKGROUND

Coccidiosis of poultry is a disease caused by protozoan parasites of the genus *Eimeria.* Oocysts of *Eimeria* species are ubiquitous in the environment and persist for many months in poultry litter. Ingestion of oocysts leads to infection of the various regions of the intestinal tract in a species-specific manner. The organism proliferates in the intestine over a period of several days, resulting in the excretion of the next generation of oocysts in the feces. Multiple cycles of infection lead to immunity, and when the infection is presented to a flock early and in a uniform dosage among the flock, the immunity developed over several cycles of exposure can be quite robust.

In contrast, when birds are not presented with the infection in a uniform manner, situations may arise in which naïve birds are subject to sudden, massive infection, leading to poor performance in terms of feed conversion and weight gain, and a high risk of secondary infections. Currently, the most common method used for control of coccidiosis in the poultry industry is not vaccination, but rather the administration of anticoccidial drugs in the feed. The low rate of vaccine use is often attributed to uncertainty in the uniformity in dosing via the feed or water at the growout facility or by spray cabinet vaccination at the hatchery, which are the traditional routes and times of administration. There is increasing interest in improving the uniformity of vaccine delivery during administration at the hatchery and thereby providing more uniform protection within the flock.

Recently, in ovo vaccination techniques have been found applicable to administration of a live oocyst-based coccidiosis vaccine (see, e.g., U.S. Pat. No. 6,500,438; U.S. Pat. No. 6,495,146; and U.S. Pat. No. 6,627,205; all to Pfizer, Inc.). The *in ovo* route of administration provides a convenient method of delivering a uniform dose of vaccine to each embryo while it is still in the egg. Delivery of avian vaccines *in ovo* is currently practiced for approximately 85% of the 9 billion broiler birds produced in the United States each year and in a growing percentage of the 21 billion broiler birds produced outside of the United States each year (see, e.g., U.S. Pat. No. 4,458,630 to the United States government). Therefore, the potential market for a live, *in ovo*-delivered coccidiosis vaccine is considerably larger than the current market for post hatch-delivered coccidiosis vaccines.

*Eimeria* oocysts contain four sporocysts within the protective oocyst wall. Sporocysts may be used for various purposes, including vaccines, viability testing, sporozoite production, etc. Conventional methods of cracking oocysts and releasing sporocysts utilize glass beads. Shaking oocysts with glass beads causes the oocyst wall to crack and release the sporocysts held therewithin. For example, Dulski et al (Avian Diseases, American Association of Avian Pathologists, vol. 32, no. 2, 1 January 1988, pages 235-239) discloses a method of releasing and purifying sporocysts and sporozoites from *Eimeria tenella* oocysts using glass bead grinding at 200 rpm to release sporocysts and using percoll density gradients to purify. Another method is described in Coudert et al (Biotechnology Guidelines on Techniques in Coccidiosis Research, 1995, pages 52 to 73), comprising a step of incubating the oocysts at 4°C in 20% sodium hypochlorite according to the method of Hosek *et al* (described below) followed by disruption with a Potter homogeniser (taking care not to use too many strokes, in order to avoid sporocyst damage). However, considerable shaking is generally required to crack a high percentage of the oocysts, and the continued shaking action may degrade previously released sporocysts. Similar problems exist with other conventional methods of releasing sporocysts, such as using a tissue grinder to release sporocysts. Sporocysts released early in the process can be destroyed by continuing the grinding process.

Other conventional methods of releasing sporocysts from oocysts involve chemically releasing sporocysts from oocysts. Typically, oocysts are suspended in a buffer containing, for example, CO₂ gas. Cysteine hydrochloride may also be included, This process reduces disulfide bonds in the micropyle region of the oocyst. Eventually, the sporocysts may be released through the loosened micropyle cap. Hosek et al (J. of Protozoology, Vol 35, No. 4, 1988 pages 583-589) describes allowing the oocycsts to sit for 24 hours at 4°C in sodium hypochlorite followed by shaking and incubating the washed oocyst pellet in an excystation solution for 1 to 2 hours at 41 °C. Unfortunately, chemical release alone is not always an efficient method for sporocyst release.

As such, conventional methods of releasing sporocysts from sporulated oocysts are inefficient and yield only a fraction of the potential viable sporocysts available. Accordingly, a need exists for improved ways of releasing sporocysts from oocysts and that overcome the problems associated with the conventional methods.

### SUMMARY

In view of the above discussion, there is provided herein a method of releasing sporocysts from oocysts, the method comprising: preparing a solution containing oocysts suspended therein; allowing a high pressure stream of said solution to collide at ultra-high velocity in a precisely defined microchannel or chamber, wherein said high pressure ranges from 1000 to 6000 psi, and wherein said microchannel or chamber is designed with fixed-geometry and is configured to accelerate a product stream to high velocities sufficient to rupture walls of the oocysts and release viable sporocysts therefrom; and recovering the released viable sporocysts from the solution. According to some embodiments of the present invention, an aqueous solution of oocysts is passed through one or more Microfluidizer® processor chamber units under defined conditions of chamber diameter, chamber geometry, and pressure. Oocysts impact the wall of the chamber and are subjected to controlled, high shear forces, tearing open the oocyst wall and releasing the sporocysts intact. This method is particularly effective in releasing sporocysts because a high percentage of oocyst walls can be cracked allowing a high percentage of sporocysts to be recovered. Moreover, little damage is done to released sporocysts allowing a high percentage of the recovered sporocysts to remain viable.

In some embodiments, the oocysts are treated to weaken the walls thereof prior to subjecting the solution to controlled shear forces. For example, the oocysts may be thermally treated, chemically treated, enzymatically treated, or may be subjected to various combinations of thermal, chemical and enzymatic treatment.

In some embodiments, the recovered sporocysts are cryopreserved for storage. In some embodiments, the recovered sporocysts are used to prepare vaccine and/or a diagnostic assay.

In some embodiments, sporozoites are excysted from the recovered sporocysts. These sporozoites may be used to prepare a vaccine and/or a diagnostic assay.

Exemplary oocysts from which sporocysts may be recovered, according to embodiments of the present invention, are *Eimeria* oocysts, such as *Eimeria* oocysts are selected from the group consisting of *E. maxima* oocysts, *E*. *mitis* oocysts, *E*. *tenella* oocysts, *E. acervulina* oocysts, *E. brunetti* oocysts, *E*. *necatrix* oocysts, *E*. *praecox* oocysts, *E. mivati* oocysts, and any combination thereof; *Eimeria* oocysts selected from the group consisting of *E*. *meleagrimitis* oocysts, *E*. *adenoeides* Oocysts, *E. gallopavonis* oocysts, *E*. *dispersa* oocysts, *E. innocua* oocysts, and *E*. *subrotunda* oocysts, and any combination thereof; *Eimeria* oocysts selected from the group consisting of *E*. *zuernii* oocysts, *E. bovis* oocysts, and any combination thereof; *Eimeria* oocysts selected from the group consisting of *E. ahsata* oocysts, *E*. *bakuensis* oocysts, *E. crandallis* oocysts, *E. faurei* oocysts, *E. granulosa* oocysts, *E. intricata* oocysts, *E. marsica* oocysts, *E. ovinoidalis* oocysts, *E. pallida* oocysts, *E. parva* oocysts, *E. weybridgensis* oocysts, and any combination thereof; and *Eimeria* oocysts selected from the group consisting of *E*. *intestinalis* oocysts, *E*. *vejdovskyi* oocysts, *E*. *piriformis* oocysts, *E*. *coecicola* oocysts, *E. irresidua* oocysts, *E. flavescens* oocysts, *E. exigua* oocysts, *E. magna* oocysts, *E. perforans* oocysts, *E. media* oocysts, *E. stiedai* oocysts, and any combination thereof.

Methods of releasing sporozoites from oocysts are provided wherein a solution of oocysts is subjected to controlled shear forces sufficient to rupture the oocyst walls and release sporozoites therefrom. An aqueous solution of oocysts is passed through one or more Microfluidizer® processor chamber units under defined conditions of chamber diameter, chamber geometry, and pressure. Oocysts impact the wall of the chamber and are subjected to controlled, high shear forces, tearing open the oocyst wall and releasing the sporozoites intact.

Embodiments of the present invention are advantageous over conventional methods. For example, embodiments of the present invention produce repeatable, consistent yields of sporocysts. In contrast, conventional glass bead and tissue grinding methods are not readily adapted to large-scale production.

### BRIEF DESCRIPTION OF THE DRAWINGS

**Figs. 1-2** are flow charts of operations for releasing sporocysts from oocysts, according to some embodiments of the present invention.
**FIG. 3** is a block diagram of a Microfluidizer® processor, according to some embodiments of the present invention.
**Fig. 4** is a flow chart of operations for processing released sporocysts, according to some embodiments of the present invention.
**Fig. 5** is a flow chart of operations for processing released sporocysts and excysting sporozoites from the released sporocysts, according to some embodiments of the present invention.

### DETAILED DESCRIPTION

The present invention now is described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art.

Like numbers refer to like elements throughout. In the figures, the thickness of certain lines, layers, components, elements or features may be exaggerated for clarity. All publications, patent applications, patents, and other references mentioned herein are incorporated herein by reference in their entireties.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items. As used herein, phrases such as "between X and Y" and "between about X and Y" should be interpreted to include X and Y. As used herein, phrases such as "between about X and Y" mean "between about X and about Y." As used herein, phrases such as "from about X to Y" mean "from about X to about Y."

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity. The sequence of operations (or steps) is not limited to the order presented in the claims or figures unless specifically indicated otherwise.

Embodiments of the present invention are suitable for releasing sporocysts from oocysts for medical and veterinary uses, as well as for diagnostic and/or research purposes. The oocysts can be from a protozoan that infects any animal subject, including mammalian and avian subjects. The terms "animal" and "animal subjects" include but are not limited to mammalian and/or avian subjects. Suitable mammalian subjects include but are not limited to primate subjects (*e.g*., human subjects and non-human primate subjects such as simian), porcine, bovine (*e.g*., cattle), caprine, equine, feline, ovine, canine, murine (*e.g*., mouse, rat) and lagomorph subjects.

The terms "avian" and "avian subjects" (*i.e*., "bird" and "bird subjects"), as used herein, are intended to include males and females of any avian species, but are primarily intended to encompass poultry that are commercially raised for eggs, meat or as pets. Accordingly, the terms "avian" and "avian subject" are particularly intended to encompass but not be limited to chickens, turkeys, ducks, geese, quail, pheasant, parakeets, parrots, cockatoo, cockatiel, ostrich, emu and the like. In particular embodiments, the avian subject is a chicken or a turkey subject. As used herein, an "avian" or "avian subject" can refer to an avian embryo *in ovo* or an avian post hatch.

The terms "cryopreserve" and "cryopreserving" are well understood by those of skill in the art of the present invention and refer to preserving cells and other material by freezing and storing at very low temperatures.

The present invention relates generally to methods of releasing sporocysts from protozoan oocysts. Such methods find use, for example, in methods of manufacturing vaccines. Many protozoa form a life stage designated as an "oocyst." The invention can be practiced to release sporocysts from oocysts of any species of protozoa containing sporocysts, including but not limited to *Eimeria, Cyclospora, Toxoplasma, Neospora and Isospora.*

The present invention may also relate to methods of releasing sporozoites from protozoan oocysts. Some protozoa form a life stage designated as an "oocyst" but may contain sporozoites within the oocyst and do not produce sporocysts. Such methods find use, for example, in methods of releasing sporozoites from oocysts including but not limited to cell-line infection, infectivity assays, manufacturing vaccines, or diagnostic assays. The invention may be practiced to release sporozoites from oocysts of any species of parasite that contains sporozoites within the oocyst, including but not limited to *Cryptosporidium and Plasmodium.*

The terms "protozoa," "oocyst," "sporocyst," "sporozoite" and "merozoite" have their accepted meanings in the art. Unless indicated otherwise, these terms are intended to refer to live (*i.e*., viable) protozoa, oocysts, sporocysts, sporozoites and merozoites, including attenuated forms, although those skilled in the art will appreciate that vaccines can be formulated using killed protozoa, oocysts, sporocysts, sporozoites and merozoites. It is understood by those skilled in the art that killed vaccines are usually prepared by first purifying the live organism. Also encompassed herein are genetically modified protozoa, oocysts, sporocysts, sporozoites and merozoites.

The term *"Eimeria"* indicates one or more species of the genus *Eimeria.* The term *"Eimeria"* includes but is not limited to strains or species of *Eimeria* that infect birds (*e.g*., chicken, turkey) or mammalian (*e.g*., cattle, sheep or rabbit) species. Such *Eimeria* species include those that are found in chickens, including, but not limited to, *E*. *tenella, E. acervulina, E. maxima, E. necatrix, E. mitis, E. praecox, E. mivati* and *E. brunetti;* and also those that are found in turkeys, including, but not limited to, *E. meleagrimitis, E. adenoeides, E. gallopavonis, E. dispersa, E. innocua,* and *E*. *subrotunda,* and those that infect cattle such as, but not limited to, *E*. *bovis* and *E. zuemii; Eimeria* species that infect sheep such as, but not limited to, *E*. *ahsata, E. bakuensis, E. crandallis, E. faurei, E. granulosa, E. intricata, E. marsica, E. ovinoidalis, E. pallida, E. parva, E. weybridgensis;* and *Eimeria* species that infect rabbits including, but not limited to, *E*. *intestinalis, E. vejdovskyi, E. piriformis, E. coecicola, E. irresidua, E. flavescens, E. exigua, E. magna, E. perforans, E. media,* and *E*. *stiedai.* In addition, the term *"Eimeria"* includes all strains of the foregoing species of *Eimeria* including, but not limited to, wildtype strains, precocious or otherwise selected strains, attenuated strains, and oocysts that have been attenuated, *e.g*., by irradiation, chemical treatment and the like. Further, the term *"Eimeria"* also includes any newly-discovered strains or species of *Eimeria.* Finally, the term *"Eimeria"* encompasses live and killed *Eimeria,* although live Eimeria are intended unless indicated otherwise.

Compositions comprising *Eimeria* oocysts find use in methods of immunizing birds against coccidiosis. Methods of vaccinating birds against coccidiosis are known in the art, and include *in ovo* (*e.g.,* U.S. Patent No. 6,500,438; U.S. Patent No. 6,495,146; and U.S. Patent No. 6,627,205; Pfizer Inc.) and post hatch (*e.g*., U.S. Patent No. 3,147,186 to Auburn Research Foundation; U.S. Patent No. 5,055,292 and U.S. Patent No. 4,438,097, both to National Research Development Corporation) vaccination methods.

The term "protozoa" includes wildtype strains, precocious or otherwise selected strains, attenuated strains, and oocysts that have been attenuated, e.g., by irradiation, chemical treatment and the like. Further, the term "protozoa" also includes any newly-discovered strains or species of protozoans. Finally, the term "protozoa" covers both live and killed protozoa, although live protozoa are intended unless indicated otherwise. The terms "produce," "producing" or "production" of oocysts, and the like generally refer to the process of harvesting oocysts from an animal and purifying the oocysts from the fecal material.

Methods of producing oocysts, such as *Eimeria* oocysts, are known in the art (*see, e.g*., U.S. Patent No. 3,147,186 to Auburn Research Foundation; U.S. Patent No. 4,544,548 to Internationale Octrooi Maatschappij "Octropa" B.V.; U.S. Patent No. 4,863,731 to Unilever Patent Holdings; international patent publications WO 00/50072 to Pfizer, Inc.; WO 03/020917 to Embrex, Inc.; and WO 02/37961 to Novus International, Inc.; Hammond et al., (1944) Amer. J. Vet. Res. 5:70; Hill et al., (1961) J. Parasit. 47:357; Jackson, (1964) Parasitology 54:87; Lotze et al., (1961) J. Parasit. 47:588; Schmatz et al., (1984) J. Protozoo/. 31:181; Whitlock, (1959) Aust. Vet. J. 35:310); Kowalik et al., (1999) Parasitol. Res. 85:496-499.

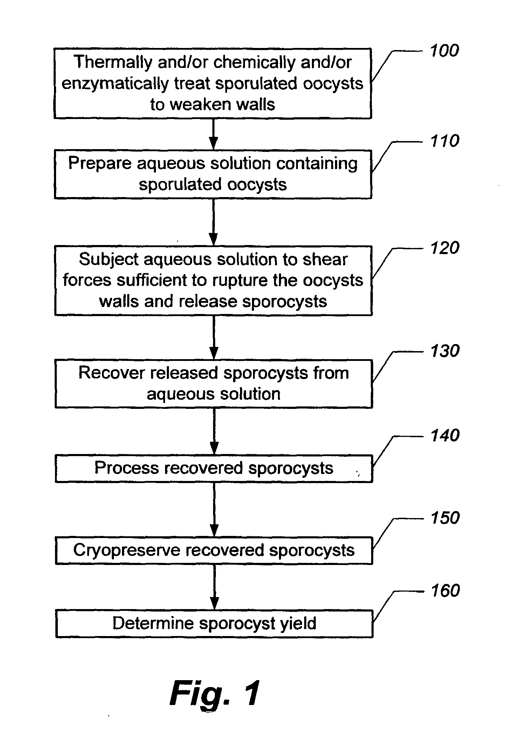

Referring to **Figs. 1-2**, methods of releasing sporocysts from sporulated oocysts, according to some embodiments of the present invention, will now be discussed. Initially, sporulated oocysts may be thermally and/or chemically and/or enzymatically pretreated to weaken the walls of the oocysts (Block **100**). The weakening caused by thermal, chemical and/or enzymatic treatment causes the oocyst walls to become more susceptible to disruption by shear forces subsequently applied thereto. Exemplary thermal treatments include, but are not limited to, heating the oocysts to between 37°C and 41°C for 0.5 hour to 2 hours. Exemplary chemical treatments include, but are not limited to, hypochlorite solution or aqueous solutions containing dissolved carbon dioxide and cysteine hydrochloride, as well as taurodeoxycholic acid. Exemplary enzymatic treatments include, but are not limited to, pepsin and various phospholipases, for example. Thermally, chemically and/or enzymatically pretreating sporulated oocysts is optional and is not required in embodiments of the present invention. Moreover, particular types of sporulated oocysts may not require pretreatment to weaken the walls thereof. Various combinations of thermal, chemical, and/or enzymatic treatment may be used.

An aqueous solution containing sporulated oocysts suspended therein is prepared (Block **110**). Exemplary aqueous solutions include, but are not limited to, Hank's balanced salt solution (HBSS), phosphate buffered saline (PBS), RPMI medium, DMEM medium, or the above solutions in combination with a protein such as casein or a protein hydrolysate such as casein hydrolysate or soy protein hydrolysate. The aqueous solution is then subjected to shear forces sufficient to rupture the oocysts' walls and release the sporocysts therefrom (Block **120**). The released sporocysts are recovered from the aqueous solution (Block **130**). The recovered sporocysts may proceed to subsequent processing (Block **140**) and/or may be cryopreserved (Block **150**).

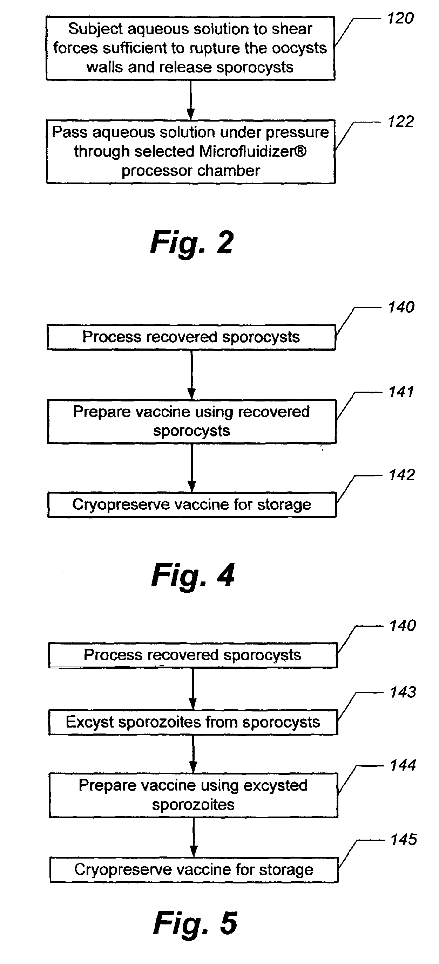

Referring to **Fig. 2**, according to some embodiments of the present invention, subjecting an aqueous solution of oocysts to shear forces sufficient to rupture the oocyst walls and release sporocysts therefrom (Block **120**) is performed by passing the aqueous solution under pressure (*e.g*., between about 2,000 psi and about 6,000 psi) through a Microfluidizer® processor chamber (Block **122**). Microfluidizer® processors are available from the Microfluidics Corporation, 30 Ossipee Road, Newton, MA. Microfluidizer® processors allow high pressure streams of solutions to collide at ultra-high velocities in precisely defined microchannels or chambers. (Other cell disruption equipment can be used including, but not limited to, the Constant Cell Disruption System produced by Constant Systems Ltd., Daventry, Northants, NN11 4SD, England, UK). Embodiments of the present invention are not limited to the use of Microfluidizer® processor chambers.

A Microfluidizer® processor chamber subjects a solution flowing therethrough to combined forces of shear and impact. Each chamber is designed with a fixed-geometry, and is configured to accelerate a product stream to high velocities. The fixed-geometry configuration allows applied shear forces to be precisely controlled and monitored.

Control of shear forces is achieved through the use of defined combinations of chamber geometry, chamber diameter, and applied pressure. Other aspects of the controlled process can include the characteristics and formulation of the solution used, including parameters such as viscosity, specific gravity, chemical composition, osmolality, pH and temperature. Optimized conditions can be determined by those of skill in the art using routine procedures. Consistency of the process may be ensured by performing test runs under defined conditions using buffer alone and determining the flow rate. Tracking the flow rate by such a test over time yields an indication of wear in the equipment or other fault in the system, and corrective action may be taken to return the system to standard operating conditions.

Exemplary Microfluidizer® processor chambers that may be used according to some embodiments of the present invention include chambers with "Y-shaped" and "Z-shaped" configurations. Y-shaped chambers include two entering flow paths that converge at a point and exit as a single flow path. Z-shaped chambers have a single flow path with a Z-shaped path. Chamber configurations for optimal recovery of sporocysts may vary by species. Accordingly, different chamber configurations may be selected for different types of oocysts. In addition, Microfluidizer® processor chambers may be arranged in series.

In addition, Microfluidizer® processor chambers may have different diameters. For example, diameters of between about 75 microns (µm) and about 500 µm may be utilized. Microfluidizer® processor chambers of different diameters may be arranged in series, also.

Applicants have found that chambers having a diameter that is substantially equal to or larger than the diameter of the oocysts in solution are particularly effective. For example, for *E. maxima* oocysts, which are typically about 20 µm to 40 µm in diameter, a reaction chamber with a diameter of about 300 µm is preferred. However, diameters ranging from about 75 µm to about 400 µm may also be used. Typically, the flow path used is the Z configuration, although a Y configuration or other configuration may also be used. Flow rates normally range from about 500 mL per min to about 2000 mL per minute at pressures ranging from 1000 to 5000 psi. The amount of shear force typically required to release sporocysts from *E. maxima* oocysts ranges from about 3.00 x 10⁵ sec⁻¹ per minute to about 1.50 x 10⁶ sec⁻¹ per-minute.

For *E*. *tenella* oocysts, which are typically about 15 µm to 25 µm in diameter, and *E. acervulina,* which are typically about 10 µm to 15 µm in diameter, a Microfluidizer® processor reaction chamber with a diameter of 100 µm is preferred. However, diameters ranging from about 75 µm to about 400 µm may also be used. Typically, the flow path used is the Z configuration, although a Y configuration or other configuration may also be used. Flow rates normally range from about 100 mL per min to about 250 mL per minute at pressures ranging from 1000 to 4000 psi. The amount of shear force typically required to release *E. tenella* or *E. acervulina* sporocysts from oocysts ranges from about 1.00 x 10⁶ sec⁻¹ per minute to about 3.00 x 10⁶ sec⁻¹ per minute.

For any species of Eimeria oocysts, conditions resulting in lower shear forces may be used to release sporocysts from oocysts, especially when oocysts have been thermally, chemically, or enzymatically pre-treated. Although multiple passes through the chamber may be used, a single pass is preferred. Embodiments of the present invention are not limited to a particular chamber diameter for a particular oocyst. Embodiments of the present invention may utilize chambers having all types of configurations and diameters.

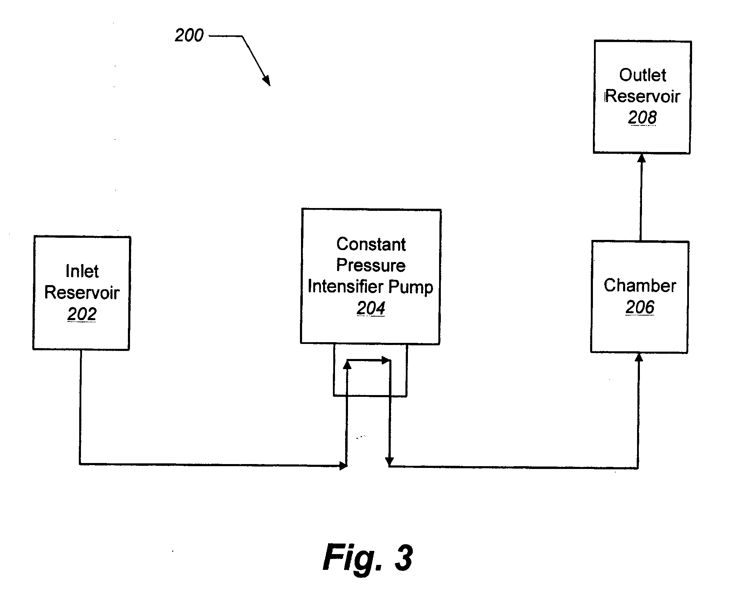

Referring to **Fig. 3**, a Microfluidizer® processor **200** is illustrated. The illustrated Microfluidizer® processor **200** includes an inlet reservoir **202** containing an aqueous solution of sporulated oocysts. The aqueous solution is pressurized and pumped via a pump **204** (*e.g*., constant pressure intensifier pump, etc.) through a chamber **206** (*e.g*., a Y-shaped, Z-shaped chamber, etc.). The oocysts in the pressurized aqueous solution are subjected to shear and impact forces in the chamber **206** causing sporocysts to be released from the oocyst walls. The aqueous solution containing the released sporocysts is collected in the outlet reservoir **208**.

Referring back to **Fig. 1**, according to some embodiments of the present invention, a percentage of sporocysts released and recovered from oocysts (*i.e*., sporocyst yield) may be determined (Block **160**). Sporocyst yield may be assessed, for example, via microscopy. Sporocysts deemed recovered by microscopy may be either viable or non-viable or a mixture thereof. However, the viability status may not be determinable by microscopy alone. Accordingly, a determination of a percentage of released sporocysts that are viable (*i.e*., viability yield) may be performed, also. Determining viability yield may require an *in vivo* procedure. Appropriate *in vivo* procedures may include administering sporocyst preparations to avian subjects via oral gavage and comparing the resulting oocyst output with that obtained by administering intact oocysts of equivalent number.

Referring to **Fig. 4**, recovered sporocysts may be processed in various ways and for various purposes. For example, vaccines may be prepared from recovered sporocysts (Block **141**) and cryopreserved for storage (Block **142**). Referring to **Fig. 5**, according to some embodiments of the present invention, recovered sporocysts may be processed to release (*i.e*., excyst) sporozoites therefrom (Block **143**). The released sporozoites may be used to prepare vaccines (Block **144**) or may be used for some other purpose. Vaccines prepared from excysted sporozoites may be cryopreserved for storage (Block **145**).

Any type of oocyst may be processed in accordance with embodiments of the present invention. Particularly suitable oocysts are *Eimeria* oocysts including, but not limited to, *E. maxima* oocysts, *E*. *mitis* oocysts, *E*. *tenella* oocysts, *E. acervulina* oocysts, *E*. *brunetti* oocysts, *E. necatrix* oocysts, *E. praecox* oocysts, *E. mivati* oocysts, and any combination thereof, *E*. *meleagrimitis* oocysts, *E. adenoeides* oocysts, *E*. *gallopavonis* oocysts, *E*. *dispersa* oocysts, *E. innocua* oocysts, and *E*. *subrotunda* oocysts, and any combination thereof, *E*. *zuemii* oocysts, *E. bovis* oocysts, and any combination thereof, *E. ahsata* oocysts, *E. bakuensis* oocysts, *E*. *crandallis* oocysts, *E*. *faurei* oocysts, *E*. *granulosa* oocysts, *E. intricata* oocysts, *E. marsica* oocysts, *E*. *ovinoidalis* oocysts, *E*. *pallida* oocysts, *E*. *parva* oocysts, *E*. *weybridgensis* oocysts, and any combination thereof, *E*. *intestinalis* oocysts, *E*. *vejdovskyi* oocysts, *E*. *piriformis* oocysts, *E*. *coecicola* oocysts, *E*. *irresidua* oocysts, *E. flavescens* oocysts, *E*. *exigua* oocysts, *E*. *magna* oocysts, *E*. *perforans* oocysts, *E. media* oocysts, *E*. *stiedai* oocysts, and any combination thereof.

Having described the present invention, the same will be explained in greater detail in the following examples, which are included herein for illustration purposes only, and which are not intended to be limiting to the invention.

### Example 1

A series of experiments were performed to map out sporocyst recovery and oocyst cracking over a broad range of parameter values. Each experiment used a total of approximately 2 x 10⁸ sporulated oocysts in 500 mL HBSS (4 x 10⁵ sporulated oocysts per mL); a sub-sample was taken for enumeration prior to Microfluidizer® processor treatment. Chamber diameters to be tested varied according to species: 1) *E. maxima:* 200, 300, 400 micron diameter chambers; 2) *E*. *tenella:* 200, 300 micron diameter chambers; 3) *E*. *acervulina:* 125 micron diameter chamber. Single pass mode was used. Pressures ranging from 2,000 psig to 6,000 psig were used, depending on the study. After passing through the Microfluidizer® processor, the total volume was adjusted to 1 L with HBSS, the sample was mixed, and a subsample was taken for enumeration. Typically, three replicate runs were made using each set of conditions.

Percent sporocysts recovered and percent oocysts cracked were determined for each experiment using hemacytometer counts. Ideally, both percent sporocysts recovered and percent oocysts cracked would be 100%.

**E. acervulina Sporocyst Release using Microfluidizer® processor**

| Chamber diameter (µm) | Pressure | | | |
|---|---|---|---|---|
| | 5000 psig | | 6000 psig | |
| | % Sporocyst Recovery | % Oocysts Cracked | % Sporocyst Recovery | % Oocysts Cracked |
| 125 Y | 54.26 | 48.18 | 49.46 | 75.61 |

Conclusions for E. acervulina 1 Microfluidizer® experiments:
1) *E. acervulina* percent oocysts cracked were observed to increase with increasing pressure using the 125 micron chamber.
2) The best results for sporocyst recovery were obtained using the 125 micron chamber at 5000 psig (54% recovery).

**E. maxima 1 sporocyst release using Microfluidizer® processor**

| Chamber Diameter (µm) | Pressure | | | | | |
|---|---|---|---|---|---|---|
| | 2000 psig | | 3000 psig | | 4000 psig | |
| | % Sporocyst Recovery | % Oocysts Cracked | % Sporocyst Recovery | % Oocysts Cracked | % Sporocyst Recovery | % Oocysts Cracked |
| 200 Z | 96.83 | 47.55 | 131.06 | 64.34 | 119.50 | 83.45 |
| 300 Z | 109.55 | 79.07 | 106.03 | 89.91 | 95.33 | 93.98 |
| 400 Z | 96.69 | 55.73 | 116.93 | 80.90 | 129.85 | 87.74 |

| | | | | | | |
|---|---|---|---|---|---|---|
| Values are averages of at least three replicate single-pass runs. | | | | | | |

Conclusions for E. maxima 1 Microfluidizer® experiments:
1) The sporocyst release process worked reasonably well for nearly all combinations of chamber diameter and pressure tested.
2) The combination of the 300 micron chamber and 3000 psig pressure provided suitable initial standard conditions for production of *E. maxima 1* sporocysts using a Microfluidizer® processor.

**E. maxima 2 Sporocyst Release using Microfluidizer® processor**

| Chamber Diameter (µm) | Pressure | | | | | |
|---|---|---|---|---|---|---|
| | 2000 psig | | 3000 psig | | 4000 psig | |
| | % Sporocyst Recovery | % Oocysts Cracked | % Sporocyst Recovery | % Oocysts Cracked | % Sporocyst Recovery | % Oocysts Cracked |
| 200 Z | 114.18 | 93.99 | 104.58 | 87.07 | 73.71 | 95.23 |
| 300 Z | 93.47 | 85.95 | 115.43 | 91.01 | 110.31 | 92.80 |
| 400 Z | 98.12 | 86.63 | 110.04 | 94.24 | 111.32 | 97.84 |

| | | | | | | |
|---|---|---|---|---|---|---|
| Values are averages of at least three replicate single-pass runs. | | | | | | |

Conclusions for *E*. *maxima* 2 Microfluidizer® processor experiments:
1) The sporocyst release process worked reasonably well for nearly all combinations of chamber diameter and pressure tested.
2) The combination of the 300 micron chamber and 3000 psig pressure was considered optimal.

**E. tenella 1 Sporocyst Release using Microfluidizer® processor**

| Chamber Diameter (µm) | Pressure | | | | | |
|---|---|---|---|---|---|---|
| | 2000 psig | | 3000 psig | | 4000 psig | |
| | % Sporocyst Recovery | % Oocyst Cracked | % Sporocyst Recovery | % Oocysts Cracked | % Sporocyst Recovery | % Oocysts Cracked |
| 125 Y | 50.33 | 54.16 | 68.46 | 71.88 | 98.18 | 84.04 |
| 200 Z | 19.86 | 13.95 | 43.80 | 39.74 | 56.30 | 62.03 |

| | | | | | | |
|---|---|---|---|---|---|---|
| Values are averages of at least three replicate single-pass runs. | | | | | | |

Conclusions for E. tenella 1 Microfluidizer® processor experiments:
*1) E. tenella* sporocyst recovery and percent oocysts cracked were observed to increase with increasing pressure for both chambers tested, as expected.
2) The best results were obtained using the 125 micron chamber at 4000 psig.

Embodiments of the present invention provide a repeatable, scalable alternative for recovery of sporocysts over conventional tissue grinder, glass bead, and chemical release methods. Essentially quantitative recovery of sporocysts from *E*. *maxima 1* and *E. maxima* 2 is provided. Nearly quantitative recovery of *E. tenella* sporocysts is observed. Recovery of *E*. *acervulina* oocysts is about 40-50%.

### Example 2

Using cell disruption equipment to release sporocysts requires optimization of release conditions to ensure viability of released sporocysts. Viability may be assessed by providing a dose of sporocysts to birds and by enumerating the associated oocyst output resulting from the infection. Under varying conditions of cell disruption equipment chamber geometry and pressure, it is possible to efficiently release sporocysts from oocysts in a non-viable condition. That is, the infection produced in birds upon administration of the sporocysts may be lessened as compared to that achieved using sporocysts released by traditional methods such as glass beads. Conditions of chamber geometry and pressure must be carefully assessed to ensure that viable sporocysts are produced.

Sporocysts were released from *E*. *acervulina* oocysts using either glass beads or the Microfiuidizer® processor configured with a 100Z chamber at 2000 psi or 5000 psi. Sporocysts released by each method were then administered to chickens in a dose response model using 1000, 3000, and 5000 sporocysts per dose. Three replicate pens were used for each treatment, with nine birds per replicate. An oocyst control at 1250 sporulated oocysts per dose representing 5000 sporocysts was also included in the test. Oocysts were collected from days 4 to 7 post gavage into a solution of 10% citric acid, 0.75% hydrogen peroxide, and 0.25% propionic acid in water. Feces containing oocysts were brought to a uniform volume using water, blended, and subsampled. Oocysts were enumerated using the McMaster's method. Results are shown in the table below:

**Effect of Pressure on the Viability of E. acervulina Sporocysts**

| Dose | Treatment | Mean Oocyst Output per Bird |
|---|---|---|
| 1250 | Sporulated oocyst control | 5.18 x 10⁷ |
| 1000 | Microfluidizer® at 2000psi | 1.22 x 10⁷ cd |
| | Microfluidizer® 5000psi | 2.85 x 10⁶ e |
| | Glass Beads | 1.58 x 10⁷c |
| 3000 | Microfluidizer® at 2000 psi | 3.93 x 10⁷b |
| | Microfluidizer® 5000psi | 6.07 x 10⁶de |
| | Glass Beads | 5.30 x 10⁷ab |
| 5000 | Microfluidizer® at 2000 psi | 1.00 x 10⁸ab |
| | Microfluidizer® 5000psi | 7.61 x 10⁶cd |
| | Glass Beads | 1.11 x 10⁸a |

| | | |
|---|---|---|
| Statistical comparisons were made between treatments receiving sporocysts. Different letters in common represent significant differences to p=.05. | | |

Results indicate that sporocysts produced using the Microfluidizer® processor at 2000psi were as viable as sporocysts produced using the traditional glass bead method at each dose, while sporocysts produced at 5000psi using the Microfluidizer® processor were significantly less viable than those produced using the glass bead method at each dose. The range of pressure which yields viable sporocysts must therefore be determined experimentally.

### Example 3

The smaller *Eimeria* species, including *E. acervulina* (∼12 micron length) and *E*. *tenella* (∼20 micron length) are observed to be less susceptible to shear than the larger *E*. *maxima* species (~40 microns length). Generating higher levels of shear by increasing the pressure in the Microfluidizer® system can improve microscopic yield of sporocysts from oocysts for any species, but is especially required for efficient release of the smaller species; however, increasing pressure can also decrease viability. Lowering pressure to improve viability inherently sacrifices yield. Pre-treatments have been developed to condition the wall of the oocysts to provide both improved microscopic yield and improved viability.

A study was performed to examine the effect of pre-treatment of oocysts using a combination of a bile salt (taurodeoxycholic acid (TDCA), an anaerobic environment (bubbled carbon dioxide), and a warm temperature (37°C for 1h). The objectives of the study were to determine the *in vitro* recovery (via microscopy) and the *in vivo* viability (via oocyst output) of released sporocysts.

The following treatment groups were established: (1) *E. acervulina* sporulated oocyst control; 1000 sporulated oocysts per dose; (2) Glass bead-produced sporocyst control; 4000 sporocysts per dose; (3) Microfluidizer®-produced sporocyst control without pretreatment; 100 µm chamber; 2,000 psi; 4000 sporocysts per dose; (4) Microfluidizer®-produced sporocysts with pretreatment including 0.75% taurodeoxycholic acid (TDCA) and bubbled carbon dioxide at 37°C for 1 h; 100 µm chamber; 2,000 psi; 4000 sporocysts per dose; (5) Microfluidizer®-produced sporocysts with pre-treatment including 0.75% TDCA and bubbled carbon dioxide at 37°C for 1h;100 µm chamber; 3,000 psi; 4000 sporocysts per dose; (6) Microfluidizer®-produced sporocysts with pretreatment including 0.75% TDCA and bubbled carbon dioxide at 37°C for 1 h; 100 µm chamber; 4,000 psi; 4000 sporocysts per dose. Residual intact oocysts and oocyst shells were removed from the sporocyst preparations using Percoll. Sporocyst doses were delivered to birds at 4,000 sporocysts per dose to provide doses equivalent to the 1,000 oocyst per dose control, as each oocyst contains four sporocysts.

Recovery of sporocysts from oocysts was evaluated microscopically. Results for the Microfluidizer®-produced materials are summarized in the table below:

**Microscopic Recovery of E. acervulina Sporocysts using Pre-Treatment**

| Treatment | Pre-Treatment | Microfluidizer® Pressure (psi) | Sporocysts Recovered (%) |
|---|---|---|---|
| 3 | No | 2000 | 5.42 |
| 4 | Yes | 2000 | 33.70 |
| 5 | Yes | 3000 | 54.90 |
| 6 | Yes | 4000 | 65.48 |

Pre-treatment improved recovery of sporocysts approximately six-fold at 2000 psi, and at increasing pressures, further improvement in microscopic recovery was observed.

For the *in vivo* assessment, each treatment used 5 replicate pens with nine birds per pen. Treatments were administered via oral gavage to the crop. Feces were collected into a solution of 10% citric acid, 0.75% hydrogen peroxide, and 0.25% propionic acid in water from days 4 to 7 post gavage. Feces containing oocysts were brought to a uniform volume, blended, and subsampled. Oocysts were enumerated using the McMaster's method.

The viability of sporocysts produced using the stated release methods was assessed by comparing oocyst output per bird relative to the glass bead-produced sporocyst control. Results are summarized in the table below:

**Viability of E. acervulina Sporocysts Released from Pre-Treated Oocvsts**

| Treatment | Eimeria Life Stage | PreTreatment | Release method | Microfluidizer® Pressure (psi) | Mean Oocyst Output per Bird | Viability as Percent of Glass Bead Control |
|---|---|---|---|---|---|---|
| 1 | Sporulated oocysts | NA | NA | NA | 3.00 x 10⁷ | NA |
| 2 | Sporocysts | NA | Glass Beads | NA | 2.97 x 10⁷ | 100.0 |
| 3 | Sporocysts | No | Microfluidizer® | 2000 | 2.58 x 10⁷ | 86.9 |
| 4 | Sporocysts | Yes | Microfluidizer® | 2000 | 4.05 x 10⁷ | 136.4 |
| 5 | Sporocysts | Yes | Microfluidizer® | 3000 | 2.41 x 10⁷ | 81.1 |
| 6 | Sporocysts | Yes | Microfluidizer® | 4000 | 4.03 x 10⁷ | 136.0 |

There were no significant differences among treatments in mean oocyst output per bird (p>.05). Pre-treatment provided improved viability of released sporocysts across a wide range of pressure. In some cases, the viability of sporocysts released from pretreated oocysts by the Microfluidizer® method was numerically higher than that of the glass bead-released sporocysts. The overall effect of pre-treatment was thus two-fold, improving both recovery from oocysts during the release step and viability.

The foregoing is illustrative of the present invention and is not to be construed as limiting thereof. Although a few exemplary embodiments of this invention have been described, those skilled in the art will readily appreciate that many modifications are possible. The invention is defined by the following claims.

## Claims

1. A method of releasing sporocysts from oocysts, the method comprising: preparing a solution containing oocysts suspended therein; allowing a high pressure stream of said solution to collide at ultra-high velocity in a precisely defined microchannel or chamber, wherein said high pressure ranges from 1000 to 6000 psi, and wherein said microchannel or chamber is designed with fixed-geometry and is configured to accelerate a product stream to high velocities sufficient to rupture walls of the oocysts and release viable sporocysts therefrom; and recovering the released viable sporocysts from the solution.

2. The method of Claim 1, wherein said fixed-geometry is a "Y-shaped" configuration or a "Z-shaped" configuration.

3. The method of Claim 1 or Claim 2, wherein said solution is passed under pressure through the chamber one or more times.

4. The method of Claim 1, wherein the solution comprises an aqueous solution.

5. The method of Claim 1, wherein the pressure used to release sporocysts from *Eimeria maxima* oocysts ranges from 1000 to 5000 psi.

6. The method of Claim 1, wherein the pressure used to release sporocysts from *E. tenella* or *E. acervulina* oocysts ranges from 1000 to 4000 psi.

7. The method of Claim 3, wherein:
(a) the chamber has a Z-shaped configuration, or
(b) the chamber has a Y-shaped configuration.

8. The method of Claim 3, wherein:
(a) a plurality of chambers is provided, and further comprising selecting a chamber having a diameter that is substantially equal to or larger than a diameter of the oocysts and passing the solution under pressure through the selected chamber, or
(b) a plurality of chambers in series is provided, each chamber having a respective different diameter, and further comprising selecting a chamber having a diameter that is substantially equal to or larger than a diameter of the oocysts and passing the solution under pressure through the selected chamber.

9. The method of Claim 1, comprising:
(a) thermally treating the oocysts to weaken the walls thereof prior to subjecting the solution to the method of claim 1, or
(b) chemically treating the oocysts to weaken the walls thereof prior to subjecting the solution to the method of claim 1, or
(c) enzymatically treating the oocysts to weaken the walls thereof prior to subjecting the solution to the method of claim 1, or
(d) using a combination of thermal, chemical, or enzymatic treatment of oocysts to weaken the walls thereof prior to subjecting the solution to the method of claim 1.

10. The method of Claim 1, further comprising cryopreserving the recovered sporocysts.

11. The method of Claim 1, further comprising preparing a vaccine and/or a diagnostic assay using the recovered sporocysts.

12. The method of Claim 1, further comprising excysting sporozoites from the recovered sporocysts.

13. The method of Claim 1, further comprising:
(a) determining a percentage of sporocysts released from the oocysts, or
(b) determining a percentage of released sporocysts that are viable.

14. The method of Claim 1, wherein the oocysts are *Eimeria* oocysts.

15. A method of releasing sporozoites from oocysts, the method comprising: preparing a solution-contalning oocysts suspended therein; allowing a high pressure stream of said solution to collide at ultra-high velocity in a precisely defined microchannel or chamber, wherein said high pressure ranges from 1000 to 6000 psi, and wherein said microchannel or chamber is designed with fixed-geometry and is configured to accelerate a product stream to high velocities sufficient to rupture walls of the oocysts and release viable sporozoites therefrom; and recovering the released viable sporozoites from the solution.

16. The method of Claim 15, wherein said fixed-geometry is a "Y-shaped" configuration or a "Z-shaped" configuration.

17. The method of Claim 15 or Claim 16, wherein said solution is passed under pressure through the chamber one or more times.

18. The method of Claim 15, wherein the solution comprises an aqueous solution.

19. The method of Claim 15, comprising treating the oocysts to weaken the walls thereof prior to subjecting the solution to the method of claim 15.

20. The method of Claim 15, further comprising cryopreserving the recovered sporozoites.

21. The method of Claim 15, further comprising preparing a vaccine and/or a diagnostic assay using the recovered sporozoites.

## Patentansprüche

1. Verfahren zur Freisetzung von Sporozysten aus Oozysten, wobei das Verfahren umfasst: Herstellen einer Lösung, die darin Oozysten suspendiert, enthält; Kollidierenlassen eines Hochdruckstroms der Lösung mit ultrahoher Geschwindigkeit in einem genau definierten Mikrokanal oder einer genau definierten Kammer, wobei der hohe Druck von 1000 bis 6000 psi reicht, und wobei der Mikrokanal oder die Kammer mit festgelegter Geometrie gestaltet ist und so konfiguriert ist, um einen Produktstrom zu ausreichend hohen Geschwindigkeiten zu beschleunigen, um Wände der Oozysten aufzureißen und lebensfähige Sporozysten daraus freizusetzen, und Gewinnen der freigesetzten lebensfähigen Sporozysten aus der Lösung.

2. Verfahren nach Anspruch 1, wobei die festgelegte Geometrie eine "Y-förmige" Konfiguration oder eine "Z-förmige" Konfiguration ist.

3. Verfahren nach Anspruch 1 oder Anspruch 2, wobei die Lösung ein- oder mehrmals unter Druck durch die Kammer geleitet wird.

4. Verfahren nach Anspruch 1, wobei die Lösung eine wässrige Lösung umfasst.

5. Verfahren nach Anspruch 1, wobei der Druck, der verwendet wird, um Sporozysten aus *Eimeria-maxima-*Oozysten freizusetzen, von 1000 bis 5000 psi reicht.

6. Verfahren nach Anspruch 1, wobei der Druck, der verwendet wird, um Sporozysten aus *E.-Tenella-* oder *E.-Acervulina-*Oozysten freizusetzen, von 1000 bis 4000 psi reicht.

7. Verfahren nach Anspruch 3, wobei:
(a) die Kammer eine Z-förmige Konfiguration hat oder
(b) die Kammer eine Y-förmige Konfiguration hat.

8. Verfahren nach Anspruch 3, wobei:
(a) eine Vielzahl von Kammern bereitgestellt wird, und es außerdem Auswählen einer Kammer, die einen Durchmesser, der im Wesentlichen gleich einem oder größer als ein Durchmesser der Oozysten ist, hat, und Durchleiten der Lösung unter Druck durch die ausgewählte Kammer umfasst, oder
(b) eine Vielzahl von Kammern in Reihe bereitgestellt wird, wobei jede Kammer einen entsprechenden anderen Durchmesser hat, und das außerdem Auswählen einer Kammer, die einen Durchmesser, der im Wesentlichen gleich einem oder größer als ein Durchmesser der Oozysten ist, hat, und Durchleiten der Lösung unter Druck durch die ausgewählte Kammer umfasst.

9. Verfahren nach Anspruch 1, umfassend:
(a) thermisches Behandeln der Oozysten, um die Wände derselben zu schwächen, bevor sie der Lösung nach dem Verfahren von Anspruch 1 unterworfen werden, oder
(b) chemisches Behandeln der Oozysten, um die Wände derselben zu schwächen, bevor sie der Lösung nach dem Verfahren von Anspruch 1 unterworfen werden, oder
(c) enzymatisches Behandeln der Oozysten, um die Wände derselben zu schwächen, bevor sie der Lösung nach dem Verfahren von Anspruch 1 unterworfen werden, oder
(d) Verwenden einer Kombination aus thermischer, chemischer oder enzymatischer Behandlung von Oozysten, um die Wände derselben zu schwächen, bevor sie der Lösung nach dem Verfahren von Anspruch 1 unterworfen werden.

10. Verfahren nach Anspruch 1, das außerdem Kryokonservieren der gewonnenen Sporozysten umfasst.

11. Verfahren nach Anspruch 1, das außerdem Herstellen eines Impfstoffs und/oder eines diagnostischen Assays unter Verwendung der gewonnenen Sporozysten umfasst.

12. Verfahren nach Anspruch 1, das außerdem Exzystieren von Sporozoiten aus den gewonnenen Sporozysten umfasst.

13. Verfahren nach Anspruch 1, außerdem umfassend:
(a) Bestimmen des Prozentgehalts an Sporozysten, die aus den Oozysten freigesetzt wurden, oder
(b) Bestimmen des Prozentgehalts von freigesetzten Sporozysten, die lebensfähig sind.

14. Verfahren nach Anspruch 1, wobei die Oozysten *Eimeria*-Oozysten sind.

15. Verfahren zur Freisetzung von Sporozoiten aus Oozysten, wobei das Verfahren umfasst: Herstellen einer Lösung, die Oozysten suspendiert darin enthält; Kollidierenlassen eines Hochdruckstroms der Lösung mit ultrahoher Geschwindigkeit in einem genau definierten Mikrokanal oder in einer genau definierten Kammer, wobei der Hochdruck von 1000 bis 6000 psi reicht und wobei der Mikrokanal oder die Kammer mit festgelegter Geometrie gestaltet ist und so konfiguriert ist, um einen Produktstrom zu ausreichend hohen Geschwindigkeiten zu beschleunigen, um Wände der Oozysten zu zerreißen und lebensfähige Sporozoiten daraus freizusetzen, und Gewinnen der freigesetzten lebensfähigen Sporozoiten aus der Lösung.

16. Verfahren nach Anspruch 15, wobei die festgelegte Geometrie eine "Y-förmige" Konfiguration oder eine "Z-förmige" Konfiguration ist.

17. Verfahren nach Anspruch 15 oder Anspruch 16, wobei die Lösung ein- oder mehrmals unter Druck durch die Kammer geleitet wird.

18. Verfahren nach Anspruch 15, wobei die Lösung eine wässrige Lösung umfasst.

19. Verfahren nach Anspruch 15, das Behandeln der Oozysten, um die Wände derselben zu schwächen, vor Unterwerfen der Lösung dem Verfahren nach Anspruch 15 umfasst.

20. Verfahren nach Anspruch 15, das außerdem Kryokonservieren der gewonnenen Sporozoiten umfasst.

21. Verfahren nach Anspruch 15, das außerdem Herstellen eines Impfstoffs und/oder eines diagnostischen Assays unter Verwendung der gewonnenen Sporozoiten umfasst.

## Revendications

1. Procédé de libération de sporocystes contenus dans des oocystes, le procédé comprenant : la préparation d'une solution contenant des oocystes en suspension à l'intérieur ; la permission d'une collision d'un flux à haute pression de ladite solution à une vitesse ultra élevée dans un microcanal ou une chambre définis de manière précise, dans lequel ladite haute pression va de 1 000 à 6 000 psi, et dans lequel ledit microcanal ou ladite chambre sont conçus selon une géométrie fixe et configurés pour accélérer un flux de produit vers des vitesses élevées suffisantes pour rompre les parois des oocystes et libérer les sporocystes viables de ceux-ci ; et la récupération des sporocystes viables libérés de la solution.

2. Procédé selon la revendication 1, dans lequel ladite géométrie fixe est une configuration « en forme de Y » ou une configuration « en forme de Z ».

3. Procédé selon la revendication 1 ou la revendication 2, dans lequel on fait passer ladite solution sous pression à travers la chambre une ou plusieurs fois.

4. Procédé selon la revendication 1, dans lequel la solution comprend une solution aqueuse.

5. Procédé selon la revendication 1, dans lequel la pression utilisée pour libérer des sporocystes contenus dans des oocystes de *Eimeria maxima* va de 1 000 à 5 000 psi.

6. Procédé selon la revendication 1, dans lequel la pression utilisée pour libérer des sporocystes contenus dans des oocystes de *E*. *tenella* ou de *E*. *acervulina* va de 1 000 à 4 000 psi.

7. Procédé selon la revendication 3, dans lequel :
a) la chambre a une configuration en forme de Z, ou
b) la chambre a une configuration en forme de Y.

8. Procédé selon la revendication 3, dans lequel :
a) une pluralité de chambres sont prévues, et comprenant en outre la sélection d'une chambre ayant un diamètre sensiblement égal ou supérieur au diamètre des oocystes et le passage de la solution sous pression à travers la chambre sélectionnée, ou
b) une pluralité de chambres en série sont prévues, chaque chambre ayant un diamètre respectif différent, et comprenant en outre la sélection d'une chambre ayant un diamètre sensiblement égal ou supérieur au diamètre des oocystes et le passage de la solution sous pression à travers la chambre sélectionnée.

9. Procédé selon la revendication 1, comprenant :
a) le traitement thermique des oocystes pour affaiblir les parois de ceux-ci avant de soumettre la solution au procédé de la revendication 1, ou
b) le traitement chimique des oocystes pour affaiblir les parois de ceux-ci avant de soumettre la solution au procédé de la revendication 1, ou
c) le traitement enzymatique des oocystes pour affaiblir les parois de ceux-ci avant de soumettre la solution au procédé de la revendication 1, ou
d) l'utilisation d'une combinaison d'un traitement thermique, chimique ou enzymatique des oocystes pour affaiblir les parois de ceux-ci avant de soumettre la solution au procédé de la revendication 1.

10. Procédé selon la revendication 1, comprenant en outre la cryoconservation des sporocystes récupérés.

11. Procédé selon la revendication 1, comprenant en outre la préparation d'un vaccin et/ou d'un essai diagnostique en utilisant les sporocystes récupérés.

12. Procédé selon la revendication 1, comprenant en outre le dékystement de sporozoïtes à partir des sporocystes récupérés.

13. Procédé selon la revendication 1, comprenant en outre :
a) la détermination du pourcentage de sporocystes libérés des oocystes, ou
b) la détermination du pourcentage de sporocystes libérés qui sont viables.

14. Procédé selon la revendication 1, dans lequel les oocystes sont des oocystes de *Eimeria.*

15. Procédé de libération de sporozoïtes contenus dans des oocystes, le procédé comprenant : la préparation d'une solution contenant des oocystes en suspension à l'intérieur ; la permission d'une collision d'un flux à haute pression de ladite solution à une vitesse ultra élevée dans un microcanal ou une chambre définis de manière précise, dans lequel ladite haute pression va de 1 000 à 6 000 psi, et dans lequel ledit microcanal ou ladite chambre sont conçus avec une géométrie fixe et configurés pour accélérer un flux de produit vers des vitesses élevées suffisantes pour rompre les parois des oocystes et libérer les sporozoïtes viables de ceux-ci ; et la récupération des sporozoïtes viables libérés de la solution.

16. Procédé selon la revendication 15, dans lequel ladite géométrie fixe est une configuration « en forme de Y » ou une configuration « en forme de Z ».

17. Procédé selon la revendication 15 ou la revendication 16, dans lequel on fait passer ladite solution sous pression à travers la chambre une ou plusieurs fois.

18. Procédé selon la revendication 15, dans lequel la solution comprend une solution aqueuse.

19. Procédé selon la revendication 15, comprenant le traitement des oocystes pour affaiblir les parois de ceux-ci avant de soumettre la solution au procédé de la revendication 15.

20. Procédé selon la revendication 15, comprenant en outre la cryoconservation des sporozoïtes récupérés.

21. Procédé selon la revendication 15, comprenant en outre la préparation d'un vaccin et ou d'un essai diagnostique en utilisant les sporozoïtes récupérés.
